(19) Europäisches Patentamt / European Patent Office / Office européen des brevets

(11) **EP 4 159 166 B1**

(12) **EUROPEAN PATENT SPECIFICATION**

(45) Date of publication and mention
of the grant of the patent:
**23.07.2025 Bulletin 2025/30**

(21) Application number: **20937542.7**

(22) Date of filing: **29.05.2020**

(51) International Patent Classification (IPC):
***A61L 27/32*** *(2006.01)*     ***A61L 27/50*** *(2006.01)*
***A61L 27/54*** *(2006.01)*     ***A61F 2/30*** *(2006.01)*

(52) Cooperative Patent Classification (CPC):
**A61L 27/54; A61F 2/30; A61L 27/32; A61L 27/50;**
A61F 2/32; A61F 2/3662; A61F 2/38; A61F 2/40;
A61F 2/42; A61F 2002/30011; A61F 2002/30322;
A61F 2002/30827; A61F 2002/30828;
A61F 2002/30831; A61F 2002/30838;     (Cont.)

(86) International application number:
**PCT/JP2020/021390**

(87) International publication number:
**WO 2021/240798 (02.12.2021 Gazette 2021/48)**

(54) **STEM FOR ARTIFICIAL JOINT**

SCHAFT FÜR KÜNSTLICHES GELENK

TIGE POUR ARTICULATION ARTIFICIELLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**05.04.2023 Bulletin 2023/14**

(73) Proprietor: **Kyocera Corporation
Kyoto-shi Kyoto 612-8501 (JP)**

(72) Inventor: **NODA, Iwao
Kyoto-shi, Kyoto 612-8501 (JP)**

(74) Representative: **TBK
Bavariaring 4-6
80336 München (DE)**

(56) References cited:
WO-A1-2005/034818       CN-A- 105 559 948
JP-A- 2001 037 792       JP-A- 2005 525 165
JP-A- 2008 073 098       JP-A- 2014 008 185
US-A1- 2009 198 344      US-A1- 2010 286 790

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
A61F 2002/30906; A61F 2002/3092;
A61F 2002/30925; A61F 2002/30929;
A61F 2002/30971; A61F 2002/30985;
A61F 2310/00407; A61F 2310/00467;
A61F 2310/00473; A61F 2310/0052;
A61F 2310/00796; A61L 2300/404

**Description**

TECHNICAL FIELD

The present disclosure relates to an artificial joint stem.

BACKGROUND OF INVENTION

Prior art which is related to this field is disclosed in US2010/286790A1 showing implant and method for coating an implant, and in US2009/0198344A1 showing metal implants.

The use of a biological implant for the treatment of both bone injuries and diseases is constantly expanding with the growth of the active population and aging population. In such a situation, a known biological implant is provided with a coating from the viewpoint of antimicrobial properties, adherence to bone, and the like.

For example, Patent Document 1 describes a coating for a medical implant, in which a part of the coating contains a bone-binding agent and an antimicrobial metal agent containing silver.

CITATION LIST

PATENT LITERATURE

Patent Document 1: JP 2011-512959 T

SUMMARY

The present disclosure provides an artificial joint stem according to claim 1.

BRIEF DESCRIPTION OF THE DRAWINGS

FIG. 1 is a schematic view illustrating an artificial joint stem according to an embodiment.

FIG. 2 is a schematic view illustrating an artificial joint stem according to an embodiment.

FIG. 3 is a schematic view illustrating an artificial joint stem according to an embodiment.

FIG. 4 is a schematic view illustrating a cross section of an artificial joint stem according to an embodiment.

FIG. 5 a schematic view illustrating a cross section of an artificial joint stem according to an embodiment.

FIG. 6 is a schematic view illustrating an artificial joint stem according to an embodiment.

FIG. 7 is a schematic view illustrating an artificial joint stem according to an embodiment.

FIG. 8 is a flowchart illustrating a method of manufacturing an artificial joint stem according to an embodiment.

FIG. 9 is a schematic view illustrating an artificial hip joint according to an embodiment.

FIG. 10 is a schematic view illustrating an artificial joint stem according to an embodiment.

FIG. 11 is a schematic view illustrating an artificial joint stem according to an embodiment.

FIG. 12 is a schematic view illustrating an artificial joint stem according to an embodiment.

FIG. 13 is a schematic view illustrating a cross section of an artificial joint stem according to an embodiment.

FIG. 14 is a schematic view illustrating an artificial joint stem according to an embodiment.

FIG. 15 is a schematic view illustrating an artificial joint stem according to an embodiment.

FIG. 16 is a schematic view illustrating an artificial joint stem according to an embodiment.

DESCRIPTION OF EMBODIMENTS

Hereinafter, one embodiment will be described in detail. Note that, unless otherwise specified in the present specification, "A to B", which represents a numerical range, means "A or more and B or less".

1. Artificial Joint Stem

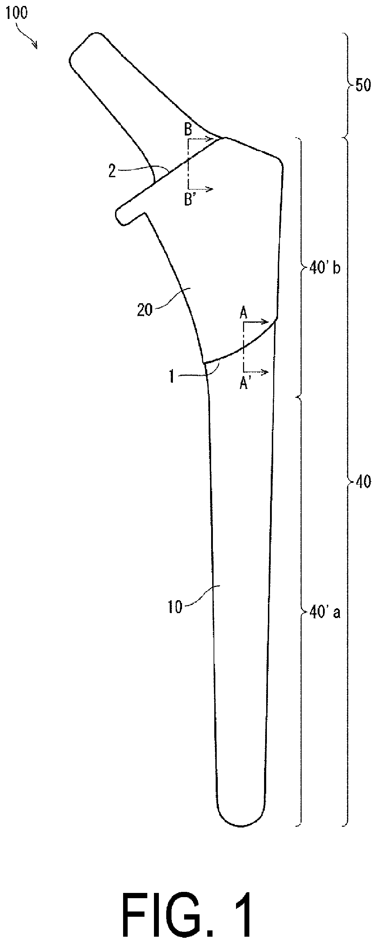

First, in an embodiment, the configuration of an artificial joint stem 100 will be described with reference to FIG. 1. The artificial joint stem 100 includes a base 10 extending in the vertical direction when the proximal side of a human body in use is defined as an upward direction, and a coating film 20 disposed on a part of the surface of the base 10. For the vertical direction of the base 10, the upward direction corresponds to the proximal direction and the downward direction corresponds to the distal direction of a human body. The coating film 20 includes a calcium phosphate-based material and an antimicrobial material. The artificial joint stem 100 includes an embedded portion 40 embedded in the bone and an exposed portion 50 exposed from the bone. In FIG. 1, of the embedded portion 40, the coating film 20 is formed in a region close to the exposed portion 50, and a region far from the exposed portion 50 is exposed from the coating film 20. Note that the calcium phosphate-based material has an effect of improving adherence to bone. The antimicrobial material is also effective in reducing bacterial adhesion and growth.

The coating film 20 is disposed on a part of the surface of the base 10. That is, the other part of the surface of the base 10 is exposed from the coating film 20. For example, it is difficult to control the adherence of the artificial joint stem 100 to bone when all of the surface of the artificial joint stem 100 is covered with a coating containing a bone-binding agent and an antimicrobial metal agent. In this case, removal of the artificial joint stem may become difficult when removal of the artificial joint stem is required after surgery. For example, the embedded portion 40 may become excessively adhered to the bone through the coating. When the base 10 includes a region covered by the coating film 20 and a region exposed from the coating film 20, excessive adhesion to the bone can be reduced. That is, the adherence to bone and the antimicrobial properties can be compatible.

The base 10 includes one or more boundary lines on the base defined by the presence or absence of the coating film 20. The one or more boundary lines include a first boundary line 1 located on the lower side of the base 10 with respect to the coating film 20. The first boundary line 1 is located so as to intersect the vertical direction. That is, all of the first boundary line 1 is not parallel to the vertical direction. A component along the vertical direction of the first boundary line 1 is smaller than a component along the width direction of the base 10.

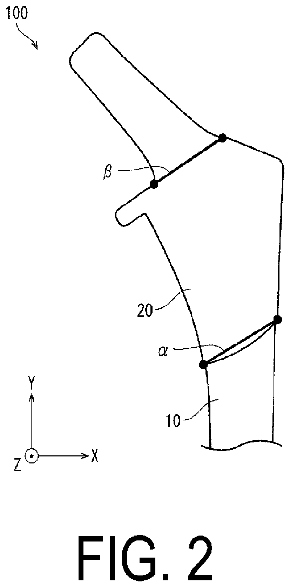

Further, the components of the first boundary line 1 will be described with reference to FIG. 2. In FIG. 2, the vertical direction of the base 10 is represented as a Y-axis direction, and the width direction of the base 10 is represented as an X-axis direction. The width direction of the base 10 may be a direction orthogonal to the vertical direction. FIG. 2 may also be a plan view from the direction perpendicular to the XY plane, that is, the Z-axis direction. In FIG. 2, a straight line $\alpha$ connecting both ends of the first boundary line 1 is examined. When the straight line $\alpha$ is parallel to the X-axis, the first boundary line 1 may contain only a component along the width direction of the base 10. When the straight line $\alpha$ is represented as a straight line having an inclination in the XY coordinates, the first boundary line 1 may contain both of a component along the vertical direction of the base 10 and a component along the width direction of the base 10. As used herein, "a component along the vertical direction of the first boundary line 1 is smaller than a component along the width direction of the base 10" means that the absolute value of the inclination of the straight line $\alpha$ is less than 1. In this case, since the friction in the shear direction occurring in the first boundary line 1 can be reduced, the peeling of the coating film 20 can be reduced. The expression "a component along the vertical direction of the first boundary line 1 is smaller than a component along the width direction of the base 10" also includes a case where the first boundary line 1 contains only a component along the width direction of the base 10.

The one or more boundary lines may include a second boundary line 2 located on the upper side of the base 10 with respect to the coating film 20. The second boundary line 2 may also be located so as to intersect the vertical direction. The second boundary line 2 may contain a component along the vertical direction smaller than a component along the width direction of the base 10, similar to the first boundary line 1. Alternatively, unlike the first boundary line 1, the component along the vertical direction of the second boundary line 2 may be greater than the component along the width direction of the base 10. In FIG. 2, a straight line connecting both ends of the second boundary line 2 is defined as a straight line $\beta$. The expression "a component along the vertical direction of the second boundary line 2 is smaller than a component along the width direction of the base 10" means that the absolute value of the inclination of the straight line $\beta$ is less than 1. On the other hand, "a component along the vertical direction of the second boundary line 2 is greater than a component along the width direction of the base 10" means that the absolute value of the inclination of the straight line $\beta$ is greater than 1. The component along the vertical direction of the second boundary line 2 may be greater than the component along the vertical direction of the first boundary line 1.

The length of the first boundary line 1 and the length of the second boundary line 2 may be the same, or one of them may be larger than the other. As used herein, the "length of the first boundary line 1 " means the total length of the first boundary line 1 that circles around the base 10. The same is true for "the length of the second boundary line 2". For example, as illustrated in FIG. 1, the length of the first boundary line 1 may be greater than the length of the second boundary line 2. On the other hand, the length of the first boundary line 1 may be smaller than the length of the second boundary line 2.

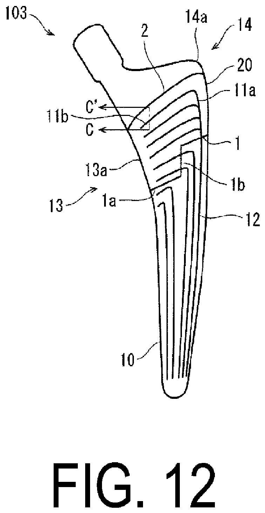

Each of the one or more boundary lines may be a straight line or a curve when the base 10 is viewed in a plan view from a direction perpendicular to the vertical direction. For example, each of the one or more boundary lines may consist of one straight line or a plurality of straight lines when the base 10 is viewed in a plan view from a direction perpendicular to the vertical direction. The expression "each of the one or more boundary lines includes a plurality of straight lines" refers to, for example, a state in which two straight lines (1a) extending in the width direction and one straight line (1b) extending in the vertical direction are connected, as illustrated in FIG. 12. Each of the one or more boundary lines may be a curve in three dimensions while including a straight line in a plan view. For example, the first boundary line 1 may consist of one straight line or may include a plurality of straight lines. The second boundary line 2 may also consist of one straight line or may include a plurality of straight lines.

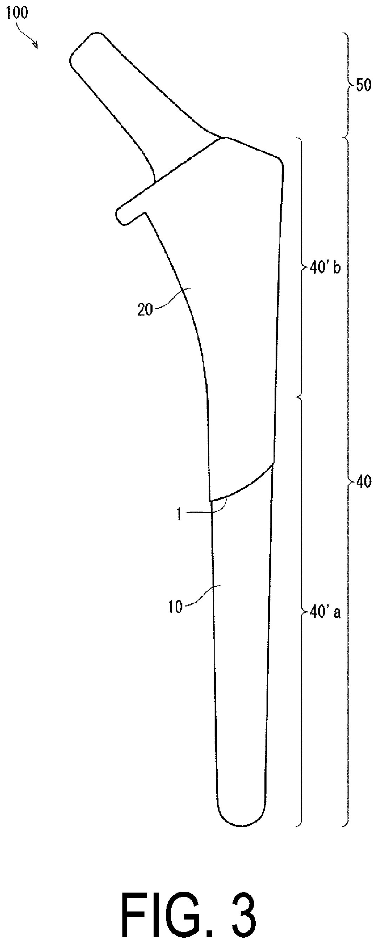

As illustrated in FIG. 1, the base 10 may include a constant width portion 40'a that is located lower in the base 10 and is constant in width. The base 10 may also include a contraction portion 40'b that decreases in width downward. The constant width portion 40'a may extend continuously downward from the contraction portion 40'b. The one or more boundary lines may be located at the constant width portion 40'a or the contraction portion 40'b. For example, the first boundary line 1 may be located at the contraction portion 40'b as illustrated in FIG. 1, or may be located at the constant width portion 40'a as illustrated in FIG. 3. The same is true for the second boundary line 2.

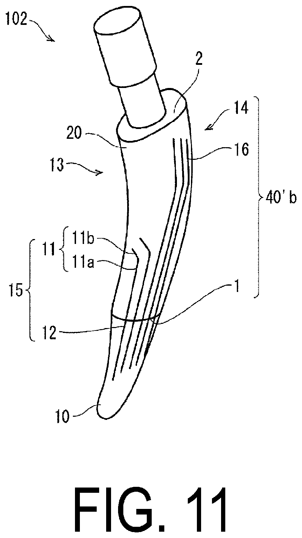

As illustrated in FIG. 11, the base 10 may include a node. The node is a portion of the base 10 where the circumference is greater than the circumference of a portion of the base 10 located above and below the node. In other words, the base 10 may be nodular. Any of the one or more boundary lines may be located at the node.

As the base 10, a metal, ceramic or plastic may be used. Examples of the metal include stainless steel alloy, cobalt-chromium alloy, titanium, and titanium alloy. The titanium alloy can be made by adding at least one selected from the group consisting of aluminum, tin, zirconium, molybdenum, nickel, palladium, tantalum, niobium, vanadium, and platinum to titanium. Examples of the ceramic include alumina, zirconia and alumina-zirconia composite ceramic. Examples of the plastic include polyethylene, fluorine-based resin, epoxy resin, polyetheretherketone (PEEK) resin, and bakelite. Note that, in the present embodiment, the base 10 is made of a titanium alloy.

The shape of the base 10 may be substantially rod-shaped, for example, but may be appropriately changed according to the shape of the artificial joint to be applied.

The coating film 20 includes a calcium phosphate-based material and an antimicrobial material. Examples of the calcium phosphate-based material include one or more types of mixtures selected from the group consisting of hydroxyapatite, α-tertiary calcium phosphate, β-tertiary calcium phosphate, quaternary calcium phosphate, octacalcium phosphate, and calcium phosphate-based glass. As the antimicrobial material, a natural antimicrobial agent, an organic antimicrobial agent, or an inorganic antimicrobial agent can be used. For example, hinokitiol can be used as a natural antimicrobial agent, benzalkonium chloride can be used as an organic antimicrobial agent, and a metal can be used as an inorganic antimicrobial agent. Examples of the metal include silver, copper, and zinc. In addition to the calcium phosphate-based material and the antimicrobial material, the coating film 20 may contain a glass ceramic, and may further contain an antimicrobial agent such as penicillin and vancomycin.

The concentration of the antimicrobial material in the coating film 20 may be, for example, from 0.05 wt% to 3.00 wt%, from 0.05 wt% to 2.50 wt%, from 0.05 wt% to 1.00 wt%, or from 0.1 wt% to 1.00 wt%. When the concentration of the antimicrobial material is 0.05 wt% or more, sufficient antimicrobial properties can be achieved. When the concentration of the antimicrobial material is 3.00 wt% or less, the impact on living tissue can be reduced.

There may be a concentration gradient of antimicrobial material in the coating film 20. For example, the concentration of the antimicrobial material contained in an upper end portion of the coating film 20 may be greater than the concentration of the antimicrobial material contained in a lower end portion of the coating film. Thus, invasion of bacteria from the upper end portion side of the coating film 20 can be more effectively reduced. The antimicrobial material may be contained only at the upper end portion of the coating film 20.

Of the surface of the base 10, the ratio between the area of a region where the coating film 20 is disposed and the area of a region where the surface of the base 10 is exposed from the coating film 20 may be appropriately determined depending on the application, or the like. The area of the region where the coating film 20 is disposed may be the same as the area of the region where the surface of the base 10 is exposed from the coating film 20, or one of the regions may be larger than the other. For example, in FIG. 1, the area of the region where the coating film 20 is disposed is smaller than the region where the surface of the base 10 is exposed from the coating film 20. In this case, the artificial joint stem 100 can be more easily inserted into the bone. On the other hand, when the area of the region where the coating film 20 is disposed is greater than the area of the region where the surface of the base 10 is exposed from the coating film 20, the invasion of bacteria can be further reduced.

The regions where the coating film 20 is disposed and the regions exposed from the coating film 20 can be distinguished by elemental analysis of the surface of each region. The method of elemental analysis can be performed, for example, by mapping the surface elements with an energy dispersive X-ray spectrometry (EDX) apparatus, which is an accessory for a general scanning electron microscope (SEM). Surface analysis methods such as X-ray photoelectron spectroscopy, Auger electron spectroscopy, and secondary ion mass spectrometry may also be used. The element may be confirmed by chemical analysis of a sample obtained by mechanically scraping off the surface of each region. For example, in the region where the coating film 20 is disposed, phosphorus, calcium, antimicrobial components, or the like are detected. On the surface of the region exposed from the coating film 20, elements constituting the base 10 are detected, and phosphorus, calcium, antimicrobial components or the like are not detected, or the detected level is below the noise level.

The base 10 may have a rough surface located on the surface. One or more boundary lines on the base 10 defined by the presence or absence of the rough surface may include a third boundary line 3 located on the lower side of the base 10 with respect to the rough surface. The one or more boundary lines on the base 10 defined by the presence or absence of the rough surface may include a fourth boundary line 4 located on the upper side of the base 10 with respect to the rough surface. The rough surface may be covered with the coating film 20 or exposed from the coating film 20. Only a part of the rough surface may be covered with the coating film 20, and the other part of the rough surface may be exposed from the coating film 20. For example, the third boundary line 3 may be located farther on the upper or lower side of the base 10 than the first boundary line 1. The fourth boundary line 4 may be located farther on the upper or lower side of the base 10 than the second boundary line 2.

An example of index of the surface roughness of the rough surface includes an arithmetic mean roughness Sa (ISO 25178). The surface roughness (Sa) of the rough surface may be set to, for example, from 10 to 80 $\mu$m, from 20 to 80 $\mu$m, or from 30 to 70 $\mu$m. Note that the surface roughness (Sa) of the rough surface can be measured, for example, by cutting the artificial joint stem 100 and observing the cut surface by SEM or the like.

The surface roughness (Sa) is only required to be measured, for example, by a stylus method or an optical method. The surface roughness (Sa) is only required to be measured in accordance with "ISO 25178", for example. Note that the measurement of the surface roughness (Sa) is not limited to the above-described method.

The artificial joint stem 100 further includes the layer member 30. As used herein, "layer member" means a member different from the coating film 20 that is layered on the base 10. For example, the surface of the layer member 30 is a rough surface. Thus, the region primarily in contact with the bone can have a rough surface. The layer member 30 may be formed by a thermal spraying method as described below. Alternatively, the layer member 30 may be formed as a porous structure.

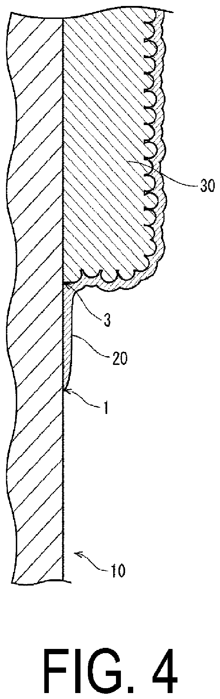

FIG. 4 illustrates a cross section A-A' including the first boundary line 1 in FIG. 1. In FIG. 4, a layer member 30 is disposed as a rough surface. Thus, the region where the layer member 30 is provided is higher than the region where the layer member 30 is not provided. Accordingly, when the artificial joint stem 100 is embedded in the bone, the layer member 30 can mainly be brought into contact with the bone. Note that in FIG. 4, the coating film 20 is formed on the layer member 30.

In FIG. 4, one or more boundary lines defined by the presence or absence of the layer member 30 as a rough surface, includes the third boundary line 3 located on the lower side of the base 10 with respect to the layer member 30. The third boundary line 3 is located farther on the upper side of the base 10 than the first boundary line 1. That is, the coating film 20 is formed so as to straddle the third boundary line 3. In this case, the edge of the layer member 30 is covered with the coating film 20. That is, the edge of the layer member 30 is covered so as not to be exposed from the coating film 20. This further reduces bacterial growth.

The third boundary line 3 may have a shape along the first boundary line 1 or may have a shape different from that of the first boundary line 1. A component along the vertical direction of the third boundary line 3 may be larger or smaller than a component along the width direction of the base 10. The components of the third boundary line 3 can be considered in the same manner as the first boundary line 1 described above.

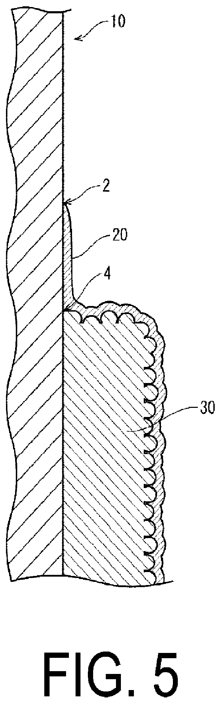

FIG. 5 illustrates a B-B' cross section including the second boundary line 2 in FIG. 1. In FIG. 5, the one or more boundary lines defined by the presence or absence of the layer member 30 as a rough surface, includes the fourth boundary line 4 located on the upper side of the base 10 with respect to the layer member 30. The fourth boundary line 4 is located farther on the lower side of the base 10 than the second boundary line 2. That is, the coating film 20 is formed so as to straddle the fourth boundary line 4. In this case, as in FIG. 4, since the edge of the layer member 30 is covered with the coating film 20, the growth of bacteria can be further reduced.

The fourth boundary line 4 may have a shape along the second boundary line 2 or may have a shape different from that of the second boundary line 2. A component along the vertical direction of the fourth boundary line 4 may be larger or smaller than a component along the width direction of the base 10. The components of the fourth boundary line 4 can be considered in the same manner as the first boundary line 1 described above.

Note that the lower limit of the height of the layer member 30 may be only required to be set to, for example, 100 $\mu$m or more, and may be set to 300 $\mu$m or more. The upper limit may be only required to be set to, for example, 1000 $\mu$m or less, and may be set to 700 $\mu$m or less. The surface roughness of the layer member 30 may be set to, for example, 10 to 80 $\mu$m, 20 to 80 $\mu$m, or 30 to 70 $\mu$m.

As the material of the layer member 30, the material exemplified as the material of the base 10 can be used. For example, the layer member 30 may be made of a metal. Thus, sufficient strength can be secured. The material of the layer member 30 and the material of the base member 10 may be the same or different. Note that in the present embodiment, the layer member 30 is made of a titanium alloy.

The height of the layer member 30 may be greater than the thickness of the coating film 20. Thus, since the region where the layer member 30 is formed is higher than the region where only the coating film 20 is formed, the region where the layer member 30 is formed can mainly be brought into contact with the bone. The thickness of the coating film 20 may be only required to be set to, for example, less than 100 $\mu$m, and may be set to less than 50 $\mu$m. The coating film 20 may be only required to be, for example, set to be 5 $\mu$m or more.

Also, as described above, the base 10 may include the embedded portion 40 embedded in the bone and the exposed portion 50 exposed from the bone. The femur is an example of the bone. A coating film 20 may be formed on a part of the peripheral wall of the embedded portion 40. Thus, the embedded portion 40, which can actually come into contact with the bone, can exhibit desirable adherence and antimicrobial properties.

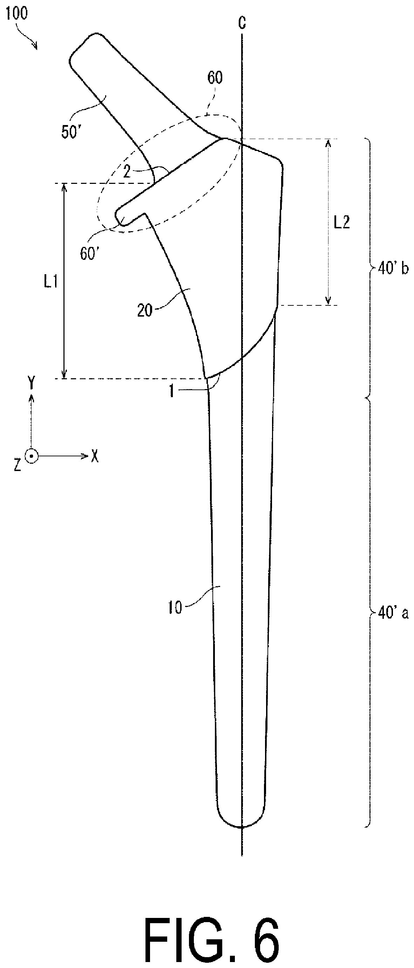

For example, as illustrated in FIG. 6, at least a part of the coating film 20 may be disposed in a boundary region 60 including a boundary between the embedded portion 40 and the exposed portion 50. That is, the coating film 20 may be disposed in a region of the embedded portion 40 near the exposed portion 50. Thus, invasion of bacteria from the exposed portion 50 side can be more effectively reduced.

The coating film 20 disposed in the boundary region 60 may be disposed only in the embedded portion 40. That is, the coating film 20 need not be disposed in the exposed portion 50. Thus, irritation to the soft tissue that may come in contact with the exposed portion 50 can be reduced.

The base 10 may include a body portion 40' including the constant width portion 40'a and the contraction portion 40'b described above, and a neck portion 50' connected to the upper end portion of the body portion 40'. The body portion 40' may be embedded in the femur. The neck portion 50' may be exposed from the femur. The neck portion 50' may be provided with a bone head. The bone head may be fitted into an acetabular cup that constitutes a pair with the artificial joint stem.

As illustrated in FIG. 6, the constant width portion 40'a may have a center axis C extending in the vertical direction. The contraction portion 40'b may extend continuously from the constant width portion 40'a in the vertical direction, and may have a shape curved such that the center of the contraction portion 40'b is separated from the center axis C as advancing upward. The contraction portion 40'b has an upper end face disposed away from the center axis C, and the neck portion 50' may be connected to the upper end face. The neck portion 50' is smaller in width than the upper end face of the body portion 40'. In other words, the neck portion 50' may be also referred to as a protruding portion 50' protruding obliquely with respect to the vertical direction of the base 10.

The base 10 may further include a collar 60' provided at a connecting portion between the body portion 40' and the neck portion 50'. The collar 60' is a protruding portion protruding from the connecting portion toward the surface direction of the upper end face. The collar 60' can reduce excessive penetration of the body portion 40' into the bone during surgery to insert the artificial joint stem into the bone.

The coating film 20 may satisfy the following relationship (1) or (2).

$$L1 \leq L2 \ (1)$$

$$L1 \geq L2 \ (2)$$

L1 ≥ L2 In the relationships (1) and (2), L1 represents the length of the coating film 20 along the vertical direction on the side where the neck portion 50' protrudes, and L2 represents the length of the coating film 20 along the vertical direction on a side opposite to the side where the neck portion 50' protrudes.

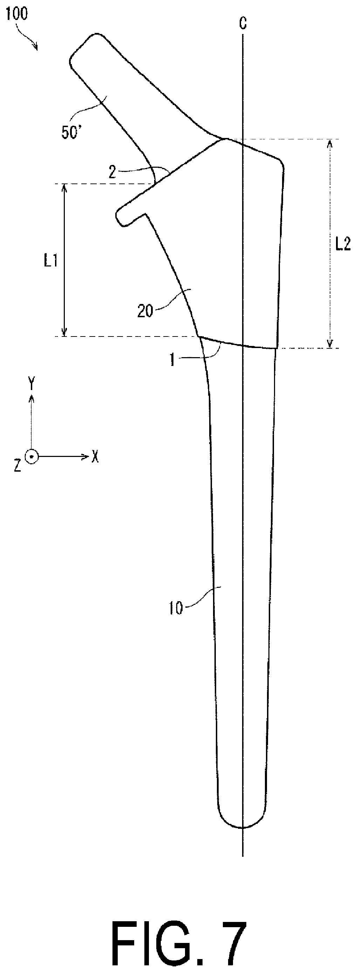

The coating film 20 illustrated in FIG. 6 satisfies the relationship (2). L1 can be also referred to as a maximum value of a length along the Y-axis direction on the side where the neck portion 50' protrudes in the region in which the coating film 20 is located. In other words, L1 represents a difference in the Y coordinate between a point where the Y coordinate of the side where the neck portion 50' protrudes in the region in which the coating film 20 is located is maximum and a point where the Y coordinate is minimum. L2 can also be referred to as a maximum value of a length along the Y-axis direction on the side opposite to the side where the neck portion 50' protrudes in the region in which the coating film 20 is located. In other words, L2 represents a difference in the Y coordinate between a point where the Y coordinate on a side opposite to the side where the neck portion 50' protrudes in the region in which the coating film 20 is located is maximum and a point where the Y coordinate is minimum. The coating film 20 illustrated in FIG. 7 satisfies the relationship (1).

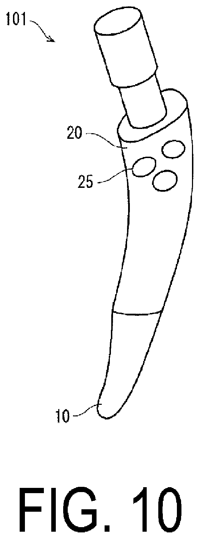

In the present disclosure, the artificial joint stem includes an artificial joint stem 101 illustrated in FIG. 10. For example, a recessed portion 25 including an opening in the surface of the coating film 20 may be provided. The opening area of the recessed portion 25 located on the upper end portion side of the coating film 20 may be greater than the opening area of the recessed portion 25 located on the lower end portion side of the coating film 20. The recessed portion 25 may be provided only at the upper end portion of the coating film 20.

In the present disclosure, the artificial joint stem also includes an artificial joint stem 102 illustrated in FIG. 11. For example, the base 10 may include a groove. The groove may straddle the region where the coating film 20 is disposed and the region exposed from the coating film 20. The groove may extend to the upper end portion of the coating film 20. The surface roughness in the groove may be smaller than that of the base 10. One end of the groove may be exposed from the coating film 20 and the other end may be located at the contraction portion 40'b of the base 10.

The base 10 may include an inner side portion 13 that curves concavely and an outer side portion 14 that curves convexly. Here, the groove may be bent at the contraction portion 40'b to either the inner side portion 13 or the outer side portion 14. For example, the groove may be bent at the contraction portion 40'b to the inner side portion 13.

The groove located in the region where the coating film 20 is disposed is defined as a first groove 11, and the groove located in the region where the surface of the base 10 is exposed from the coating film 20 is defined as a second groove 12. The first groove 11 may or may not be connected to the second groove 12. That is, the first groove 11 and the second groove 12 may be formed as a single continuous groove. The upper end portion of the first groove 11 may be located at a bend on the inner side portion side. The upper end portion of the first groove 11 may be bent to the inner side portion 13. In other words, the first groove 11 may include a first portion 11a extending in the vertical direction of the base 10 and a second portion 11b connected to the first portion 11a and having a component along the width direction of the base 10. The depth of the second groove 12 may be smaller than the depth of the first groove 11. The fact that the second portion 11b of the first groove 11 has a component along the width direction of the base 10 indicates that the second portion 11b of the first groove 11 is not parallel to the vertical direction of the base 10. In other words, the second portion 11b of the first groove 11 is not parallel to, but is inclined with respect to the bone insertion direction of the artificial joint stem 102. When the second portion 11b of the first groove 11 has a component along the width direction of the base 10, sinking can be avoided.

The base 10 may include a plurality of grooves. The base 10 may also include a first groove set 15 and a second groove set 16. The first groove set 15 includes the first groove 11 and the second groove 12 connected to each other. The second groove set 16 includes the first groove 11 and the second groove 12 connected to each other, and the first groove 11 extends to the upper end portion of the coating film 20 further than the first groove set 15.

In addition, the base 10 may include a plurality of first groove sets 15. The plurality of the first groove sets 15 may be arranged in the width direction of the base 10. Of the plurality of the first groove sets 15, the first groove set 15 located on the outer side portion 14 side may be located above the first groove set 15 located on the inner side portion 13 side.

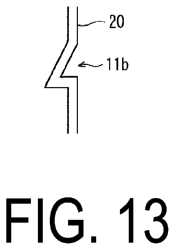

In the present disclosure, the artificial joint stem also includes an artificial joint stem 103 illustrated in FIG. 12. For example, the base 10 may include a plurality of grooves, and a groove located on the upper portion of the base 10 may be wider than a groove located on the lower portion of the base 10. The grooves may have a component along the width direction of the base 10. FIG. 13 illustrates a C-C' cross section of FIG. 12. As illustrated in FIG. 13, a groove (the second portion 11b of the first groove 11) along the width direction of the base 10 may be shallower upward.

The first boundary line 1 may intersect a linear portion of the groove. Here, the first boundary line 1 may diagonally intersect the linear portion of the groove. That is, the first boundary line 1 need not be orthogonal to the linear portion of the groove.

The first boundary line 1 may include a first portion 1a extending in a direction intersecting the groove and a second portion 1b extending in a direction along the groove. The second portion 1b of the first boundary line 1 may be separated from the groove. That is, the second portion 1b of the first boundary line 1 need not be in contact with the groove. The same is true for the second boundary line 2.

The first groove 11 and the second groove 12 are connected. The first groove 11 may include the first portion 11a extending in the vertical direction of the base 10 and the second portion 11b connected to the first portion 11a and having a component along the width direction of the base 10. As illustrated in FIG. 12, the second boundary line 2 may extend in a direction along the second portion 11b of a first groove 111.

As illustrated in FIG. 12, the second boundary line 2 may be disposed so as to incline upward from the inner side portion 13 toward the outer side portion 14. As illustrated in FIG. 12, the first boundary line 1 may be located below an apex 13a of the recessed portion at the inner side portion 13. A first boundary line 2 may be located below an apex 14a of the protruding portion at the outer side portion 14. Alternatively, the first boundary line 1 may be located above the apex 14a of the protruding portion at the outer side portion 14.

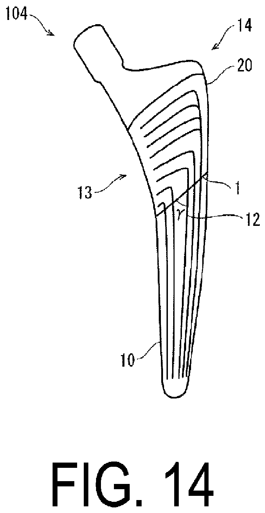

In the present disclosure, the artificial joint stem also includes an artificial joint stem 104 illustrated in FIG. 14. For example, each of the one or more boundary lines defined by the presence or absence of the coating film 20 may intersect the groove with an acute angle. In FIG. 14, an angle $\gamma$ on the inner side portion 13 side of the angle formed by the first boundary line 1 and the second groove 12 is an acute angle.

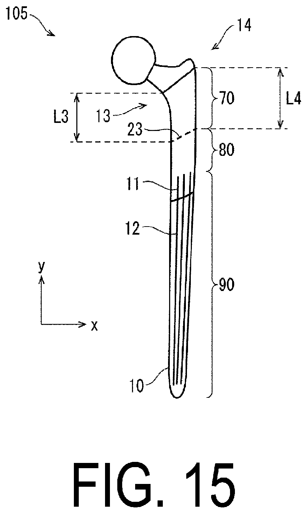

In the present disclosure, the artificial joint stem also includes an artificial joint stem 105 illustrated in FIG. 15. For example, the base 10 includes, from top to bottom, a rough surface region 70, a non-rough-surface region 80, and a groove region 90. A rough surface is disposed in the rough-surface region 70. The non-rough-surface region 80 is a region without a rough surface. The grooves are disposed in the groove region 90. For example, the rough-surface region 70 may be a region in which rough surfaces are disposed but no grooves are disposed. The non-rough-surface region 80 may be a region in which neither a rough surface nor a groove is disposed. The groove region 90 may be a region in which a rough surface is not disposed but a groove is disposed. The coating film 20 may cover at least one selected from the group consisting of the rough-surface region 70, the non-rough-surface region 80 and the groove region 90. The area of the rough-surface region 70 may be smaller than that of the non-rough-surface region 80. In the width direction of the base 10, the length of the rough-surface region 70 may be greater than the length of the non-rough-surface region 80. One or more boundary lines 23 between the rough-surface region 70 and the non-rough-surface region 80 may be inclined upward from the inner side portion 13 toward the outer side portion 14. A length L3 on the inner side portion 13 side of the rough-surface region 70 may be less than the length L4 on the outer side portion 14 side of the rough-surface region 70. Here, when viewed in a plan view from a direction perpendicular to the XY plane of FIG. 15, the length L3 represents a difference in the Y coordinate between a point where the Y coordinate is maximum and a point where the Y coordinate is minimum, on the inner side portion 13 side of the rough-surface region 70. A length L4 represents a difference in the Y coordinate between a point where the Y coordinate is maximum and a point where the Y coordinate is minimum, on the outer side portion 14 side of the rough-surface region 70.

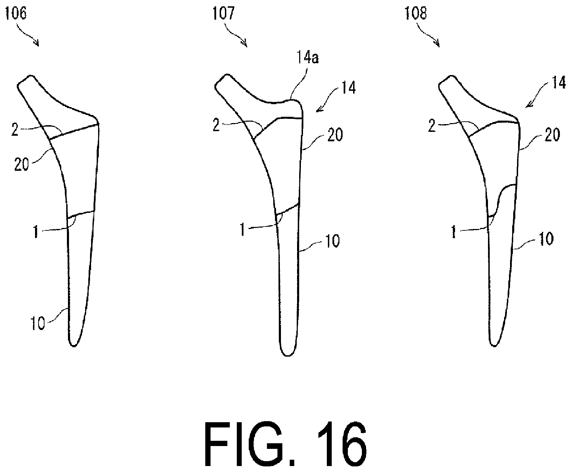

In the present disclosure, the artificial joint stem also includes artificial joint stems 106, 107, and 108 illustrated in FIG. 16. For example, the second boundary line 2 may be a straight line, such as the artificial joint stem 106. As in the artificial joint stem 107, the apex 14a of the protruding portion may be exposed from the coating film 20 at the outer side portion 14. As in the artificial joint stem 108, the second boundary line 2 may follow the shape of the outer side portion 14 of the base 10.

2. Method of Manufacturing Artificial Joint Stem

In an embodiment, a method of manufacturing an artificial joint stem includes, for example, a preparation step and a coating film forming step. In the preparation step, a base 10 having a surface including a first region and a second region is prepared. The coating film forming step is a step of forming the coating film 20 containing a calcium phosphate-based material and an antimicrobial material on a part (the first region) of the surface of the base 10. Here, the base 10 includes thereon one or more boundary lines defined by the presence or absence of the coating film 20. Of the one or more boundary lines, the first boundary line 1 is located on the lower side of the base 10 with respect to the coating film 20. The coating film 20 is formed such that the first boundary line 1 intersects the vertical direction and a component along a vertical direction of a first boundary line 1 becomes smaller than a component along a width direction of the base 10. Thus, the artificial joint stem 100 described above can be obtained.

In the preparation step, the base 10 can be prepared by molding the metal material into a desired shape by using a metal mold, or an additive manufacturing method, or the like. Note that since the second region is located so as to sandwich the first region vertically in the base 10, the first boundary line and the second boundary line can be formed after the coating film 20 is formed. The shapes of the first boundary line and the second boundary line of the coating film can be adjusted according to the shape of the first region.

The coating film 20 can be formed by: a thermal spraying method such as flame spraying, high-speed flame spraying, and plasma spraying; a physical vapor deposition method or chemical vapor deposition method such as sputtering, ion plating, ion beam deposition, and an ion mixing method; or a wet coating method such as a sol-gel method. Note that the material constituting the coating film is also referred to as a coating material.

A first protective material may be used to form the coating film 20 only in the first region. In this case, the coating film forming step may further include a step of disposing the first protective material so as to protect the second region while exposing the first region so that the coating film is not formed in the second region. For example, a masking tape or a screen may be used as the first protective material. Alternatively, a jig covering the base 10 may be used as the first protective material. Examples of the first protective material include metals, glasses, resins, and composite materials thereof. Note that the first protective material may or may not be in contact with the base 10. When, for example, a masking tape is used as the first protective material, the first protective material may be disposed on the second region. When a jig covering the base 10 is used, the shape of the jig is not particularly limited, but may be, for example, tubular. The cross section of the tubular jig may be polygonal or circular.

When a screen is to be disposed, the coating film 20 can be formed in a specific region by placing the screen in a predetermined location. When a jig is used, the coating film 20 can be formed in a specific region by placing the jig in a predetermined location. In this case, for example, the coating film 20 can be selectively formed only in a desired region by adjusting the positional relationship between the screen and a discharge nozzle configured to discharge the thermal spraying material, the additive manufacturing material, the chemical etching material, the blasting material, or the coating material. In this case, a tip of the discharge nozzle is only required to be disposed, for example, in a straight line with the surface of the desired region without being separated by the screen. Hereinafter, a thermal spraying material, an additive manufacturing material, a chemical etching material, a blasting material, or a coating material discharged from the discharge nozzle is also referred to as a discharge material. Without being limited to the above, the coating film 20 may be formed while the base 10, the screen, and the discharge nozzle are fixed, or the coating film 20 may be formed while moving at least one selected from the group consisting of the aforementioned. The angle of the discharge nozzle may be fixed or the coating film 20 may be formed while changing the angle.

Note that the coating film 20 can be formed only in a desired region without using a protective material. For example, the coating film 20 can be selectively formed only in a desired region by adjusting the shape, angle degree, or position, of the discharge nozzle configured to discharge the discharge material. For example, the discharge material may be discharged with the discharge nozzle located above the surface of the desired region. In this case, the coating film 20 may be formed by fixing the base 10 and moving the position and angle of the discharge nozzle, or the coating film 20 may be formed by fixing the discharge nozzle and moving the position and angle of the base 10. The discharge nozzle may be moved at a constant speed or at a variable speed. The discharge direction of the discharge material may be an angle of 90 ° or an angle of less than 90 ° with the vector extending from the tip of the discharge nozzle toward the base 10 or the surface of the rough surface, which are located at the shortest distance from the tip of the discharge nozzle.

A roughening step may be performed before the coating film forming step. The roughening step is a step of forming a rough surface on the base 10. Specifically, in the preparation step, the base 10 having a surface further including a roughening region in addition to the first region and the second region is prepared. Then, in the roughening step, a rough surface is formed in the roughening region of the base 10.

In the roughening step, a rough surface can be formed by at least one selected from the group consisting of a thermal spraying method, an additive manufacturing method, a chemical etching method, and a blasting method. Compared with the blasting method, the thermal spraying method, the additive manufacturing method or the chemical etching method can increase the surface roughness. Note that the material discharged toward the base 10 in the thermal spraying method is referred to as a thermal spraying material. Similarly, a material discharged toward the base 10 in the additive manufacturing method is referred to as an additive manufacturing material. A material discharged toward the base 10 when processing by the chemical etching method is referred to as a chemical etching material. Similarly, a material discharged toward the base 10 when processing by the blasting method is referred to as a blasting material. As the thermal spraying material and the additive manufacturing material, the material exemplified as the material for the base 10 can be used. The layer member described above may be formed by a thermal spraying method or an additive manufacturing method. Examples of the chemical etching method include alkali treatment. Examples of the blasting method include sandblasting.

A second protective material may be used to form a rough surface only in a desired region. In this case, the roughening step may further include a step of disposing the second protective material so as to protect other regions while exposing the roughening region so that no rough surface is formed in other than the roughening region. Note that the roughening region may be located inside the first region or may straddle across the first region and the second region. That is, the second protective material may be disposed to protect a part of the first region and the second region, or the second region. For example, the positional relationship between the third boundary line and the fourth boundary line defined by the presence or absence of the rough surface, and between the first boundary line and the second boundary line defined by the presence or absence of the coating film, can be adjusted according to the location of the roughening region.

For example, a masking tape or a screen may be used as the second protective material. Alternatively, a jig covering the base 10 may be used as the second protective material. Examples of the second protective material include metals, glasses, resins, and composite materials thereof. Note that the second protective material may or may not be in contact with the base 10. When a jig covering the base 10 is used as the second protective material, the shape of the jig is not particularly limited, but may be, for example, tubular. The cross section of the tubular jig may be polygonal or circular.

When a screen is to be placed, a rough surface can be formed in a specific region by placing the screen in a predetermined position. When a jig is to be used, a rough surface can be formed in a specific region by placing the jig in a predetermined position. In this case, for example, the rough surface can be selectively formed only in a desired region by adjusting the positional relationship between the screen and a discharge nozzle for discharging the thermal spraying material, the additive manufacturing material, the chemical etching material, the blasting material, or the coating material. In this case, a tip of the discharge nozzle is only required to be disposed, for example, in a straight line with the surface of the desired region without being separated by the screen. Hereinafter, a thermal spraying material, an additive manufacturing material, a chemical etching material, a blasting material, or a coating material discharged from the discharge nozzle is also referred to as a discharge material. Without being limited to the above, the rough surface may be formed while the base 10, the screen, and the discharge nozzle are fixed, or the rough surface may be formed while moving at least one selected from the group consisting of the aforementioned. The angle of the discharge nozzle may be fixed or the rough surface may be formed while changing the angle. Note that similarly to the coating film 20, a rough surface may be formed only in a desired region without using a protective material.

As described above, when forming a rough surface on the artificial joint stem, for example, in the roughening step, while exposing a part of the surface of the base 10, the second protective material may be disposed on the base 10 so as to protect another part of the base 10, so that a rough surface may be formed on the exposed part of the surface. In the present disclosure, the manufacturing method may further include a step of removing the second protective material after the roughening step and before the coating film forming step.

After the second protective material is removed, a step of scraping the edge of the rough surface, for example, the edge of the layer member 30, may be performed. Thus, the concentration of stress at the edge of the layer member 30 can be avoided, and irritation to the biotissue can be reduced.

A second masking tape may be used as the second protective material. In this case, in the present disclosure, the manufacturing method may further include a step of, while exposing a part of the base 10, sticking the second masking tape to another part of the base 10, before the roughening step.

In the coating film forming step, while exposing a part of the surface of the base 10, the first protective material may be disposed on the base 10 so as to protect another part of the base 10, so that the coating film 20 may be formed on the exposed part of the surface. The first protective material can be removed after the coating film forming step. A first masking tape may be used as the first protective material. In this case, in the present disclosure, the manufacturing method may further include a step of, while exposing a part of the base 10, attaching a second masking tape to another part of the base 10, before the coating film forming step.

As the second protective material, one having higher thermal resistance than that of the first protective material can be used. For example, a material which is not melted or pyrolyzed for 1 minute under a thermal spraying condition of 8000°C may be used as the second protective material, and a material which is not melted or pyrolyzed for 1 minute under a thermal spraying condition of 3000°C may be used as the first protective material. A specific example of such a material includes a composite material of glass and resin.

When processing by the blasting method in the roughening step, a material which is not dissolved or pyrolyzed at room temperature may be used as the second protective material. A specific example of such a material includes a resin.

In the present disclosure, in the step of forming the rough surface or the coating film 20 in the manufacturing method, a protective material may be disposed in addition to the first protective material and the second protective material. For example, in the step of forming the rough surface or the coating film 20, a protective material may be disposed on a part or all of the exposed portion 50. Thus, the presence or absence of the formation of the rough surface or the coating film 20 in the exposed portion 50 can be appropriately controlled. For example, by disposing a protective material in a region of the exposed portion 50 far from the embedded portion 40 and forming the coating film 20 in the exposed region, the coating film 20 can be formed in a region of the exposed portion 50 near the embedded portion 40.

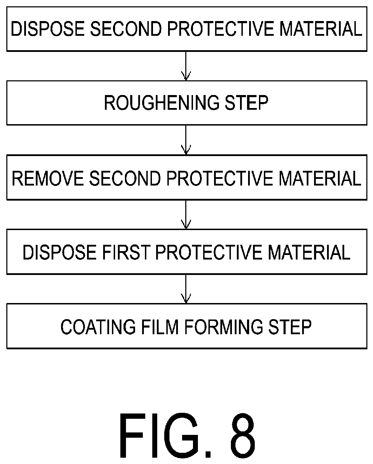

In summary, each step can be performed in the order illustrated in FIG. 8, for example. FIG. 8 is a flowchart illustrating a method of manufacturing an artificial joint stem 100 according to an embodiment. First, the second protective material is disposed, and after a roughening step is performed, the second protective material can be removed. Thereafter, the first protective material may be disposed, followed by a coating film forming step.

In the present disclosure, the manufacturing method may or may not include a cleaning step between each step. For example, in the present disclosure, the manufacturing method includes a step of cleaning the base 10, or the base 10 and the layer member 30 after the roughening step. The cleaning method is not particularly limited, but may be, for example, a method of immersing in a liquid such as water or an organic solvent such as alcohol, or a method of showering using the liquid. Alternatively, a method of blowing a gas such as air, nitrogen or argon may be employed. Thus, the excess thermal spraying material or the like and/or scrapes or the like generated by the roughening step can be removed.

In the manufacturing method according to the present disclosure, after the roughening step for roughening the surface of the base 10, a groove forming step for forming a groove may be performed, and then the coating film forming step for forming a coating film may be performed. Alternatively, after the groove forming step, the roughening step may be performed, and then the coating film forming step may be performed. In the groove forming step, the groove can be formed by at least one selected from the group consisting of a cutting method, a rolling processing method and a pressing method. For example, the groove may be formed by milling as one of the cutting methods. When forming the recessed portion, the recessed portion forming step may be performed in place of the groove forming step by the same method as the groove forming step.

The roughening step may include, in order, a first roughening step of forming a first rough surface by a thermal spraying method and a second roughening step of forming a second rough surface by a chemical etching method or a blasting method. Herein, a region where the first rough surface is formed by thermal spraying method is referred to as a first roughening region, a region where the second rough surface is formed by chemical etching method or blasting method is referred to as a second roughening region, and a region where no rough surface is formed is referred to as a non-roughening region.

In the first roughening step, a third protective material (the second protective material described above) may be disposed to protect the second roughening region and the non-roughening region with respect to the base 10 while exposing the first roughening region, thereby forming the first rough surface in the exposed first roughening region. In the present disclosure, the manufacturing method may further include a step of removing the third protective material after the first roughening step and before the second roughening step. In the second roughening step, a fourth protective material may be disposed to protect the non-roughening region while exposing the second roughening region with respect to the base 10 to form the second rough surface on the exposed second roughening region. The second roughening step may be performed such that the surface of the second rough surface formed in the second roughening step has a surface roughness smaller than that of the first rough surface in the first roughening region. In the present disclosure, the manufacturing method may include a step of removing the fourth protective material after the second roughening step and before the coating film forming step. A fourth masking tape may be used as the fourth protective material. In the present disclosure, the manufacturing method may further include a step of attaching a fourth masking tape to a non-roughening region 3 while exposing a first roughening region 1 and a second roughening region 2 before the second roughening step. As the fourth protective material, a material having lower thermal resistance than the above-described third protective material may be used. For example, a material which is not dissolved or pyrolyzed at room temperature may be used as the fourth protective material. Specifically, a resin may be used as the fourth protective material.

In the second roughening step, the non-roughening region may or may not be covered with a protective material. The first roughening region and the non-roughening region may be protected, and only the second roughening region may be processed by at least one selected from the group consisting of chemical etching method and blasting method. Alternatively, a fourth protective material may be disposed so as to expose the first roughening region, and the rough surface of the exposed first roughening region and the second roughening region may be processed by at least one selected from the group consisting of a chemical etching method and a blasting method. Thus, an excess thermal spraying material or the like remaining on the rough surface of the first roughening region can be removed, and a rough surface can be also formed on the second roughening region.

The method of manufacturing the artificial joint stem 100 may initially include a step of preparing a base 10 in which the first roughening region, the second roughening region, and the non-roughening region are located in this order.

### 3. Use of Artificial Joint Stem

Although the artificial joint stem 100 illustrated in FIG. 1 has a shape mainly assuming a stem for an artificial hip joint, artificial joints to which the artificial joint stem according to the present disclosure is applied, are not limited to artificial hip joints. Examples of the artificial joint include an artificial hip joint, an artificial knee joint, an artificial ankle joint, an artificial shoulder joint, an artificial elbow joint and an artificial finger joint.

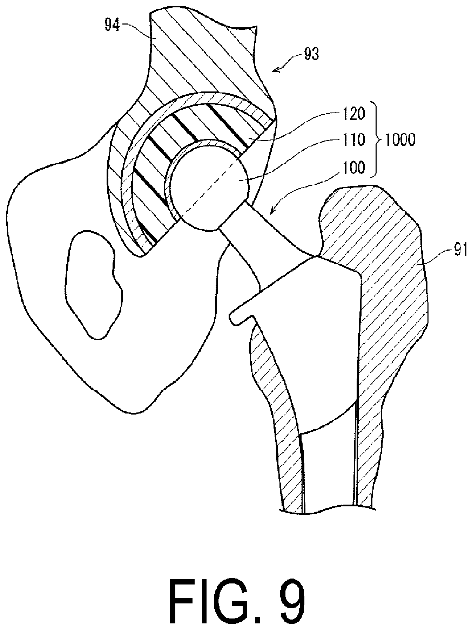

Referring now to FIG. 9, an example in which the artificial joint stem 100 is used as a part of an artificial hip joint 1000 will be described below. The artificial hip joint 1000 may include a bone head 110 and an acetabular cup 120 in addition to the artificial joint stem 100. The bone head 110 and the acetabular cup 120 may be formed of the same or different materials as the base 10 of the artificial joint stem 100. The artificial joint stem 100 is embedded in a femur 91. The bone head 110 is disposed at the exposed portion 50 of the artificial joint stem 100. The acetabular cup 120 is fixed to an acetabular 94 of a hip bone 93. The acetabular cup functions as a hip joint by fitting and sliding the bone head 110 into the depression of the acetabular cup 120.

In the present disclosure, the invention has been described above based on the various drawings and examples. However, the invention according to the present disclosure is not limited to each embodiment described above. That is, the embodiments of the invention according to the present disclosure can be modified in various ways within the scope of the claims.

REFERENCE SIGNS

1 First boundary line
2 Second boundary line
3 Third boundary line
4 Fourth boundary line
10 Base
20 Coating film
40'a Constant width portion
40'b Contraction portion
50' Neck portion
100 Artificial joint stem

### Claims

1. An artificial joint stem (100) comprising:

   a base (10) extending in a vertical direction when a proximal side of a human body in use is defined as an upward direction;
   a coating film (20) on a part of the base (10), containing a calcium phosphate-based material and an antimicrobial material; and
   one or more boundary lines (1, 2) on the base (10) defined by a presence or absence of the coating film (20), and comprising a first boundary line (1) located on a lower side of the base (10) with respect to the coating film (20), and that intersects the vertical direction, wherein
   a component along the vertical direction of the first boundary line (1) is smaller than a component along a width direction of the base (10),
   **characterized in that**
   the artificial joint stem (100) further comprising:
   a layer member (30) having a rough surface located on a surface of the base (10):

      one or more boundary lines (3, 4) on the base (10) defined by a presence or absence of the rough surface, and comprising a third boundary line (3) that is located on a lower side of the base (10) with respect to the rough surface,
      wherein the third boundary line (3) is located farther on an upper side of the base (10) than the first boundary line (1), and
      the coating film (20) is formed as to straddle the third boundary line (3) such that an edge of the layer member (30) is covered with the coating film (20).

2. The artificial joint stem (100) according to claim 1, wherein

the one or more boundary lines further comprise a second boundary line (2) located higher in the base (10) with respect to the coating film (20), and that intersects the vertical direction, and a component along the vertical direction of the second boundary line (2) is smaller than a component along the width direction.

3. The artificial joint stem (100) according to claim 2, wherein a length of the first boundary line (1) is smaller than a length of the second boundary line (2).

4. The artificial joint stem (100) according to any one of claims 1 to 3, wherein at least one boundary line of the one or more boundary lines (1, 2, 3, 4) comprising a plurality of straight lines when the base (10) is viewed in a plan view from a direction perpendicular to the vertical direction.

5. The artificial joint stem (100) according to any one of claims 1 to 3, wherein the first boundary line (1) consists of one straight line when the base (10) is viewed in a plan view from a direction perpendicular to the vertical direction.

6. The artificial joint stem (100) according to any one of claims 1 to 5, wherein

the base (10) comprises a contraction portion (40'b) decreasing in width downward, and at least one boundary line of the one or more boundary lines (1, 2, 3, 4) is located at the contraction portion (40'b).

7. The artificial joint stem (100) according to any one of claims 1 to 5, wherein

the base (10) comprises a constant width portion (40'a) that is located lower in the base (10) and is constant in width, and at least one boundary line of the one or more boundary lines (1, 2, 3, 4) is located at the constant width portion (40'a).

8. The artificial joint stem (100) according to claim 1, wherein the third boundary line (3) has a shape along the first boundary line (1).

9. The artificial joint stem (100) according to claim 1, wherein the third boundary line (3) has a shape different from the first boundary line (1).

10. The artificial joint stem (100) according to claim 9, wherein a component along the vertical direction of the third boundary line (3) is greater than a component along the width direction of the base (10).

11. The artificial joint stem (100) according to any one of claims 1, 9 to 10, wherein

the one or more boundary lines (1, 2) on the base (10) defined by a presence or absence of the coating film (20) comprise the second boundary line (2) located on an upper side of the base (10) with respect to the coating film (20), the second boundary line (2) intersects the vertical direction, the one or more boundary lines (3, 4) on the base (10) defined by a presence or absence of the rough surface comprise a fourth boundary line (4) located on an upper side of the base (10) with respect to the rough surface, and the fourth boundary line (4) is located farther on a lower side of the base (10) than the second boundary line (2).

12. The artificial joint stem (100) according to claim 11, wherein the fourth boundary line (4) has a shape along the second boundary line (2).

13. The artificial joint stem (100) according to any one of claims 1 to 12, wherein

the base (10) comprises a neck portion (50') protruding obliquely with respect to the vertical direction, and the coating film (20) satisfies the following relationship (1):

$$L1 \leq L2 \ (1),$$

where L1 represents a length of the coating film (20) along the vertical direction on a side where the neck portion (50') protrudes, and L2 represents a length of the coating film (20) along the vertical direction on a side opposite to the side where the neck portion (50') protrudes.

**Patentansprüche**

1. Schaft eines künstlichen Gelenks (100), mit:

einer Basis (10), die sich in einer vertikalen Richtung erstreckt, wenn eine proximale Seite des menschlichen Körpers im Einsatz als eine Aufwärtsrichtung definiert ist;

einem Beschichtungsfilm (20) auf einem Teil der Basis (10), der ein Material auf Calciumphosphatbasis und ein antimikrobielles Material enthält; und

eine oder mehrere durch Präsenz oder Fehlen des Beschichtungsfilms (20) definierte Grenzlinien (1, 2) auf der Basis (10), die eine erste Grenzlinie (1) aufweisen, die auf einer unteren Seite der Basis (10) in Bezug auf den Beschichtungsfilm (20) angeordnet ist, und die die vertikale Richtung kreuzt, wobei

ein Bestandteil entlang der vertikalen Richtung der ersten Grenzlinie (1) kleiner als ein Bestandteil entlang einer Breitenrichtung der Basis (10) ist,

**dadurch gekennzeichnet, dass**

der Schaft eines künstlichen Gelenks (100) ferner umfasst:

ein Schichtelement (30) mit einer rauen Oberfläche, die sich auf einer Oberfläche der Basis (10) befindet,

eine oder mehrere durch Präsenz oder Fehlen der rauen Oberfläche definierte Grenzlinien (3, 4) auf der Basis (10), die eine dritte Grenzlinie (3) aufweisen, die auf einer unteren Seite der Basis (10) in Bezug auf die raue Oberfläche angeordnet ist,

wobei die dritte Grenzlinie (3) weiter auf einer oberen Seite der Basis (10) als die erste Grenzlinie (1) angeordnet ist, und

der Beschichtungsfilm (20) so ausgebildet ist, dass die dritte Grenzlinie (3) derart gespreizt ist, dass eine Kante des Schichtelements (30) mit dem Beschichtungsfilm (20) bedeckt ist.

2. Schaft eines künstlichen Gelenks (100) nach Anspruch 1, wobei

die eine oder mehreren Grenzlinien ferner eine zweite Grenzlinie (2) umfasst, die in Bezug auf den Beschichtungsfilm (20) höher in der Basis (10) angeordnet ist, und die die vertikale Richtung kreuzt, und

ein Bestandteil entlang der vertikalen Richtung der zweiten Grenzlinie (2) kleiner als ein Bestandteil entlang der Breitenrichtung ist.

3. Schaft eines künstlichen Gelenks (100) nach Anspruch 2, wobei

eine Länge der ersten Grenzlinie (1) kleiner als eine Länge der zweiten Grenzlinie (2) ist.

4. Schaft eines künstlichen Gelenks (100) nach einem der Ansprüche 1 bis 3, wobei

zumindest eine Grenzlinie von der einen oder mehreren Grenzlinien (1, 2, 3, 4) eine Vielzahl von geraden Linien umfasst, wenn die Basis (10) in einer Draufsicht von einer Richtung senkrecht zu der vertikalen Richtung betrachtet wird.

5. Schaft eines künstlichen Gelenks (100) nach einem der Ansprüche 1 bis 3, wobei

die erste Grenzlinie (1) aus einer geraden Linie besteht, wenn die Basis (10) in einer Draufsicht von einer Richtung senkrecht zu der vertikalen Richtung betrachtet wird.

6. Schaft eines künstlichen Gelenks (100) nach einem der Ansprüche 1 bis 5, wobei

die Basis (10) einen Kontraktionsabschnitt (40'b) umfasst, der in der Abwärtsrichtung in der Breite abnimmt, und

zumindest eine Grenzlinie von der einen oder mehreren Grenzlinien (1, 2, 3, 4) sich an dem Kontraktionsabschnitt (40'b) befindet.

7. Schaft eines künstlichen Gelenks (100) nach einem der Ansprüche 1 bis 5, wobei

die Basis (10) einen konstanten Breitenabschnitt (40'a) umfasst, der sich unten in der Basis (10) befindet, und konstant in der Breite ist, und

zumindest eine Grenzlinie von der einen oder mehreren Grenzlinien (1, 2, 3, 4) sich an dem konstanten Breitenabschnitt (40'a) befindet.

8. Schaft eines künstlichen Gelenks (100) nach Anspruch 1, wobei

die dritte Grenzlinie (3) eine Form entlang der ersten Grenzlinie (1) aufweist.

9. Schaft eines künstlichen Gelenks (100) nach Anspruch 1, wobei

die dritte Grenzlinie (3) eine Form aufweist, die sich von der ersten Grenzlinie (1) unterscheidet.

10. Schaft eines künstlichen Gelenks (100) nach Anspruch 9, wobei

ein Bestandteil entlang der vertikalen Richtung der dritten Grenzlinie (3) größer als ein Bestandteil entlang der Breiterichtung der Basis (10) ist.

11. Schaft eines künstlichen Gelenks (100) nach einem der Ansprüche 1, 9 bis 10, wobei

die eine oder mehrere durch Präsenz oder Fehlen des Beschichtungsfilms (20) definierten Grenzlinien (1, 2) auf der Basis (10) die zweite Grenzlinie (2) umfassen, die auf einer oberen Seite der Basis (10) in Bezug auf den Beschichtungsfilm (20) angeordnet ist,

die zweite Grenzlinie (2) die vertikale Richtung kreuzt,

die eine oder mehrere durch Präsenz oder Fehlen der rauen Oberfläche definierten Grenzlinien (3, 4) auf der Basis (10) eine vierte Grenzlinie (4) umfassen, die auf einer oberen Seite der Basis (10) in Bezug auf die raue Oberfläche angeordnet ist, und

die vierte Grenzlinie (4) weiter auf einer unteren Seite der Basis (10) als die zweite Grenzlinie (2) angeordnet ist.

12. Schaft eines künstlichen Gelenks (100) nach Anspruch 11, wobei

die vierte Grenzlinie (4) eine Form entlang der zweiten Grenzlinie (2) aufweist.

13. Schaft eines künstlichen Gelenks (100) nach einem der Ansprüche 1 bis 12, wobei

die Basis (10) einen Nackenabschnitt (50') umfasst, der in Bezug auf die vertikale Richtung schräg hervorsteht, und

der Beschichtungsfilm (20) die folgende Beziehung (1) erfüllt:

$$L1 \leq L2 \ (1),$$

wobei

L1 eine Länge des Beschichtungsfilms (20) entlang der vertikalen Richtung auf einer Seite darstellt, an der der Nackenabschnitt (50') hervorsteht, und L2 eine Länge des Beschichtungsfilms (20) entlang der vertikalen Richtung auf einer Seite darstellt, die der Seite gegenüberliegt, an der der Nackenabschnitt (50') hervorsteht.

**Revendications**

1. Tige d'articulation artificielle (100) comprenant :

une base (10) s'étendant dans une direction verticale lorsqu'un côté proximal d'un corps humain en utilisation est défini comme étant orienté vers le haut ;

un film de revêtement (20) sur une partie de la base (10), contenant un matériau à base de phosphate de calcium et un matériau antimicrobien ; et

une ou plusieurs lignes de délimitation (1, 2) sur la base (10) définies par la présence ou l'absence du film de revêtement (20), et comprenant une première ligne de délimitation (1) située sur un côté inférieur de la base (10) par rapport au film de revêtement (20), et qui croise la direction verticale, dans laquelle

une composante le long de la direction verticale de la première ligne de délimitation (1) est plus petite qu'une composante le long d'une direction de largeur de la base (10),

**caractérisé en ce que**

la tige d'articulation artificielle (100) comprend en outre :

un élément de couche (30) ayant une surface rugueuse située sur une surface de la base (10) ;

une ou plusieurs lignes de délimitation (3, 4) sur la base (10) définie/définies par la présence ou l'absence de la surface rugueuse, et comprenant une troisième ligne de délimitation (3) qui est située sur un côté inférieur de la base (10) par rapport à la surface rugueuse,

dans laquelle la troisième ligne de délimitation (3) est située plus loin sur un côté supérieur de la base (10) que la première ligne de délimitation (1), et

le film de revêtement (20) est formé de manière à chevaucher la troisième ligne de délimitation (3) de telle sorte qu'un bord de l'élément de couche (30) soit recouvert par le film de revêtement (20).

2. Tige d'articulation artificielle (100) selon la revendication 1, dans laquelle

la une ou les plusieurs lignes de délimitation (1, 2) comprennent en outre une deuxième ligne de délimitation (2) située plus haut dans la base (10) par rapport au film de revêtement (20), et qui croise la direction verticale,

et

une composante le long de la direction verticale de la deuxième ligne de délimitation (2) est plus petite qu'une composante le long de la direction de la largeur.

3. Tige d'articulation artificielle (100) selon la revendication 2, dans laquelle

une longueur de la première ligne de délimitation (1) est plus petite qu'une longueur de la deuxième ligne de délimitation (2).

4. Tige d'articulation artificielle (100) selon l'une quelconque des revendications 1 à 3, dans laquelle

au moins une ligne de délimitation parmi la une ou les plusieurs lignes de délimitation (1, 2, 3, 4) comprend une pluralité de lignes droites lorsque la base (10) est observée dans une vue en plan depuis une direction perpendiculaire à la direction verticale.

5. Tige d'articulation artificielle (100) selon l'une quelconque des revendications 1 à 3, dans laquelle la première ligne de délimitation (1) est constituée d'une ligne droite lorsque la base (10) est observée dans une vue en plan depuis une direction perpendiculaire à la direction verticale.

6. Tige d'articulation artificielle (100) selon l'une quelconque des revendications 1 à 5, dans laquelle

la base (10) comprend une partie de contraction (40'b) dont la largeur diminue vers le bas, et au moins une ligne de délimitation parmi la une ou les plusieurs lignes de délimitation (1, 2, 3, 4) est située à la partie de contraction (40'b).

7. Tige d'articulation artificielle (100) selon l'une quelconque des revendications 1 à 5, dans laquelle

la base (10) comprend une partie de largeur constante (40'a) qui est située plus bas dans la base (10) et dont la largeur est constante, et au moins une ligne de délimitation parmi la une ou les plusieurs lignes de délimitation (1, 2, 3, 4) est située à la partie de largeur constante (40'a).

8. Tige d'articulation artificielle (100) selon la revendication 1, dans laquelle la troisième ligne de délimitation (3) a une forme qui suit la première ligne de délimitation (1).

9. Tige d'articulation artificielle (100) selon la revendication 1, dans laquelle la troisième ligne de délimitation (3) a une forme différente de celle de la première ligne de délimitation (1).

10. Tige d'articulation artificielle (100) selon la revendication 9, dans laquelle une composante le long de la direction verticale de la troisième ligne de délimitation (3) est supérieure à une composante le long de la direction de la largeur de la base (10).

11. Tige d'articulation artificielle (100) selon l'une quelconque des revendications 1, 9 à 10, dans laquelle

la une ou les plusieurs lignes de délimitation (1, 2) sur la base (10) définies par la présence ou l'absence du film de revêtement (20) comprennent la deuxième ligne de délimitation (2) située sur un côté supérieur de la base (10) par rapport au film de revêtement (20), la deuxième ligne de délimitation (2) croise la direction verticale,

la une ou les plusieurs lignes de délimitation (3, 4) sur la base (10) définies par la présence ou l'absence de la surface rugueuse comprennent une quatrième ligne de délimitation (4) située sur un côté supérieur de la base (10) par rapport à la surface rugueuse, et la quatrième ligne de délimitation (4) est située plus loin sur un côté inférieur de la base (10) que la deuxième ligne de délimitation (2).

12. Tige d'articulation artificielle (100) selon la revendication 11, dans laquelle la quatrième ligne de délimitation (4) a une forme qui suit la deuxième ligne de délimitation (2).

13. Tige d'articulation artificielle (100) selon l'une quelconque des revendications 1 à 12, dans laquelle

la base (10) comprend une partie de col (50') faisant saillie obliquement par rapport à la direction verticale, et le film de revêtement (20) satisfait à la relation suivante (1) :

$$L1 \leq L2 \ (1),$$

où
L1 représente une longueur du film de revêtement (20) le long de la direction verticale sur un côté où la partie de col (50') fait saillie, et L2 représente une longueur du film de revêtement (20) le long de la direction verticale sur un côté opposé au côté où la partie de col (50') fait saillie.

# FIG. 1

FIG. 2

# FIG. 3

# FIG. 4

# FIG. 5

FIG. 6

# FIG. 7

DISPOSE SECOND PROTECTIVE MATERIAL

↓

ROUGHENING STEP

↓

REMOVE SECOND PROTECTIVE MATERIAL

↓

DISPOSE FIRST PROTECTIVE MATERIAL

↓

COATING FILM FORMING STEP

# FIG. 8

# FIG. 9

FIG. 10

# FIG. 11

# FIG. 12

FIG. 13

# FIG. 14

# FIG. 15

# FIG. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010286790 A1
- US 20090198344 A1
- JP 2011512959 T